(19)

Europäisches
Patentamt

European
Patent Office

Office européen
des brevets

(11) **EP 4 197 442 A1**

(12) **EUROPEAN PATENT APPLICATION**

(43) Date of publication:
**21.06.2023 Bulletin 2023/25**

(21) Application number: **21214583.3**

(22) Date of filing: **15.12.2021**

(51) International Patent Classification (IPC):
**A61B 6/00** *(2006.01)*

(52) Cooperative Patent Classification (CPC):
**A61B 6/5217; A61B 6/486; A61B 6/507**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **WISSEL, Tobias
  Eindhoven (NL)**
• **KRÖNKE, Sven
  Eindhoven (NL)**

(74) Representative: **Philips Intellectual Property &
Standards
High Tech Campus 52
5656 AG Eindhoven (NL)**

(54) **PERIPHERAL PERFUSION ANALYSIS**

(57)    The present invention relates to peripheral perfusion analysis. In order to provide improved information for a perfusion analysis, a device (10) for peripheral perfusion analysis is provided. The device comprises a data input (12), a data processor (14) and an output interface (16). The data input is configured to provide a sequence of 2D angiograms (18); and to provide a measured perfusion signal (20) of perfused contrast agent in the region of interest. The data processor is configured to extract the region of interest; to define a blood supply section comprising blood flow entry into and blood flow exit out of the region of interest; to segment the blood supply section to extract regions occupied by detectable vessels; to identify vessels responsible for an inflow to extract the blood inlet into the region of interest; and to sum up all blood inlet of the section providing a perfusion sum. The output interface is configured to deconvolve the perfusion signal of the region of interest with the perfusion sum.

Fig. 1

**Description**

FIELD OF THE INVENTION

The present invention relates to a device for peripheral perfusion analysis, to a medical imaging system and to a method for peripheral perfusion analysis.

BACKGROUND OF THE INVENTION

The analysis of pre- and post-interventional foot perfusion from angiographic sequences provides information about changes in downstream perfusion after treating upstream lesions. This may be achieved by comparing contrast-density curves as a function of time, e.g. in form of time-density curve (TDC), for a region-of-interest (ROI) in a foot angiogram. The resulting TDCs vary in their shape characteristics influenced by several factors. Among those, a factor of interest is the amount and timing of the blood supply, i.e. perfusion for that region. The more and the quicker oxygenated blood reaches the tissue areas, the better metabolism can be restored, and ischemia be mitigated. Even though shape characteristics can be visually compared between two curves or quantitatively via informative parameter sets after fitting a parameterized model to it, it has been shown that it may be challenging to retrieve information in a reliable manner. WO 2005/104936 A1 relates to obtaining data perfusion measurements.

SUMMARY OF THE INVENTION

There may thus be a need to provide improved information for a perfusion analysis.

The object of the present invention is solved by the subject-matter of the independent claims; further embodiments are incorporated in the dependent claims. It should be noted that the following described aspects of the invention apply also for the device for peripheral perfusion analysis, for the medical imaging system and for the method for peripheral perfusion analysis.

According to the present invention, a device for peripheral perfusion analysis is provided. The device comprises a data input, a data processor and an output interface. The data input is configured to receive a sequence of 2D angiograms, and to receive a measured perfusion signal of perfused contrast agent in the region of interest. The input can provide the data to the data processor. The data processor is configured to extract the region of interest from the received data. The data processor is also configured to define a blood supply section comprising blood flow entry into and blood flow exit out of the region of interest. The data processor is configured to segment the blood supply section to extract regions occupied by detectable vessels. The data processor is also configured to identify vessels responsible for an inflow to extract the blood inlet into the region of interest, and to sum up all blood inlet of the section providing a perfusion sum. The data processor is also configured to deconvolve the perfusion signal of the region of interest with the perfusion sum. The output interface is configured to provide the deconvolution result.

As an effect, a region of interest related perfusion signal is put in relation with the actual blood supply and is thus normalized for an improved analysis.

According to an example, by the deconvolving, the measured contrast density signals are standardized to perfusion kernels.

According to an example, for the identification, inflow and outflow of blood are determined for the detected vessels.

According to an example, the region of interest of the subject belongs to at least one of the group of a foot, a hand and a head of a subject.

According to an example, the identification of the vessels with the inflow extracts an arterial input function (AIF) for the respective angiogram. The AIF extraction is provided covering regions of the image plane within a foot silhouette, hand silhouette or head silhouette.

According to an example, the AIF covers all inlets to the perfusion region of interest (ROI).

As an alternative option, the AIF covers the outlets. In an option, the AIF covers at least a part of the outlets. In an option, all of the outlets are considered.

According to an example, the blood supply section is provided as a boundary layer enclosing an area as the region of interest.

According to an example, the blood supply section is provided as a convex form enclosing the region of interest.

According to an example, starting from the defined blood supply section, growing sub-portions are determined and an approximation index is provided to reflect possible ambiguity due to an increasing distance of the perfusion parts from the blood supply section.

According to an example, the blood supply section is provided orthogonal to a longitudinal direction of the tibia bone at the lower end of the tibia bone.

According to an example, a display is provided. The deconvolving comprises generating an outcome in form of at least one of the group of parameter and curves. The outcome is provided for clinical evaluation. As an option, the outcome is displayed on the display.

According to the present invention, also a medical imaging system is provided. The system comprises an X-ray imaging device configured for generating X-ray images of a region of interest. The X-ray images comprise contrast-injected images and non-injected images. The system further comprises a device for peripheral perfusion analysis according to one of the preceding examples. The X-ray imaging device generates the X-ray images for the sequence of 2D angiograms.

According to the present invention, also a method for peripheral perfusion analysis is provided. The method comprises the following steps:

- receiving or obtaining a sequence of 2D angiograms;
- extracting the region of interest;
- defining a blood supply section comprising blood flow entry into and blood flow exit out of the region of interest;
- segmenting the blood supply section to extract regions occupied by detectable vessels;
- identifying vessels responsible for an inflow to extract the blood inlet into the region of interest;
- summing up all blood inlet of the section providing a perfusion sum;
- providing a measured perfusion signal of perfused contrast agent in the region of interest;
- deconvolving the perfusion signal of the region of interest with the perfusion sum; and
- providing the deconvolution result.

As an effect, standardized perfusion kernels are provided for analyzing arbitrary regions in peripheral angiograms of the foot.

A field of use is minimally invasive perfusion monitoring.

The present solution facilitates an analysis of the situations, e.g. the time density curves (TDCs), and, in particular, a comparison between pre- and post-intervention TDCs of the same patient or of TDCs from a patient population. The solution eliminates the dependencies of the contrast curve shape on factors that are not related to the body's ability to perfuse a certain foot region, i.e. the injection protocol or changing time racing effects of different components of the contrast flow, by taking the supply characteristics of a certain region into account.

In an example, existing deconvolution techniques are provided that can be leveraged to deconvolve acquired TDCs into standardized perfusion kernels which are eligible for such comparisons.

According to an aspect, the proposed solutions are dedicated to automatically extract the AIF for peripheral angiograms as a prerequisite for deconvolving measured contrast density signals in the foot and to standardize them to perfusion kernels; a contrast density response that one would expect for a short (dirac) impulse at the input. In an example, this is achieved while taking the following requirements into account: The region for AIF extraction must only cover regions of the image plane within the foot silhouette. Further, the convolution operation is associative and commutative, i.e. there is no need for the AIF to be connected to the perfusion ROI, but the AIF must cover all inlets to the perfusion ROI, because deconvolution cannot account for a complete loss of signal components. Furthermore, the AIF ignores all outlets, which are likely to overlap with the inlets.

According to an aspect, knowledge of the AIF specific for each ROI is provided, thereby being able to also provide deconvolutions.

In an option for automatically obtaining the AIF, the detection of inflow events on the margin of arbitrary ROIs is provided as a more generalized form of the following. In an option for automatically obtaining the AIF, the detection of the tibia condyles and exploiting knowledge of the local anatomy to define the AIF for ROIs bordering the ankle is provided. Both options rely on the extraction of macrovascular activity on 2D angiograms and can be used to evaluate local effects on tissue perfusion by comparing the kernels or a chosen parameterization of them.

According to an aspect, as a result of the AIF correction, perfusion kernels of the present solution will look dissimilar to those measured without the AIF correction. This stems from the fact, that the natural input to the foot vascular system is typically not a dirac impulse. With higher acquisition rates, the difference becomes more visible. Moreover, one characteristic of the perfusion kernel is that the time of arrival, i.e. start of the rising edge of the TDC, is always at time zero for arbitrary perfusion ROIs provided they directly border the AIF extraction region, because deconvolution with a delayed AIF will compensate for the time of arrival. This is not the case for non-deconvolved signals.

The method can be a computer implemented method either or not embodied in a computer program and either or not stored on a computer readable medium. It may be performed by a system having the input for receiving data, a processor for processing the data and an output for providing result data. The data may be received by the input. Alternatively, or additionally, the processor can be configured to cause the input to receive the data. Likewise, the method can comprise the step of causing the receipt of the data. This may be referred to as obtain the data under control of the processor or method whereas receipt of data may also simply include passive receipt of data without control by the processor or method. The processor is communicatively coupled to the input and output to provide data received by the input to the processor and to allow the processor to provide result data at the output to e.g. another system, device or user etc.

These and other aspects of the present invention will become apparent from and be elucidated with reference to the embodiments described hereinafter.

BRIEF DESCRIPTION OF THE DRAWINGS

Exemplary embodiments of the invention will be described in the following with reference to the following drawings:

Fig. 1 schematically shows an example of a device for peripheral perfusion analysis.
Fig. 2 shows an example of a medical imaging system.
Fig. 3 shows steps of an example of a method for peripheral perfusion analysis.
Fig. 4a and Fig. 4b show a first example of an extraction of an arterial input function.

Fig. 5a and Fig. 5b show a second example of an extraction of an arterial input function.

Fig. 6 shows an example of a graph of determined perfusion in a selected region of interest of Fig. 5a.

Fig. 7 shows different curves for different kernels for the region of interest.

Fig. 8 shows an example of growing valid perfusion regions of interest for the example of Fig. 5a.

Fig. 9a shows a first example of a mask image for a perfusion image shown in Fig. 9b.

Fig. 10 shows an example of a graph indicating a macrovascular response within the arterial input function of the first example of Fig. 9a and Fig. 9b.

Fig. 11 shows an example of a graph indicating a microvascular response within the rest of the foot of the first example of Fig. 9a and Fig. 9b.

Fig. 12a shows a second example of a mask image for a perfusion image shown in Fig. 12b

Fig. 13 shows an example of a graph indicating a macrovascular response within the arterial input function of the second example of Fig. 12a and Fig. 12b.

Fig. 14 shows an example of a graph indicating a microvascular response within the rest of the foot of the second example of Fig. 12a and Fig. 12b.

Fig. 15a shows an example of an inlet extraction module for the example of Fig. 9a as binary mask with four components.

Fig. 15b shows a digital subtraction angiography (DSA) maximum intensity projection (MIP) image relating to Fig. 15a.

Fig. 15c shows a Hough transform being used to detect lines, i.e. vessel components within the binary overall mask.

Fig. 15d shows an example for each of the final components being subject to an individual comparison with a reference flow vector to detect the set of inlet components.

Fig. 16 shows an example of a graph indicating the AIF in a deconvolution process.

Fig. 17 shows a graph indicating the perfusion signal as well as the perfusion kernel.

Fig. 18 shows curves relating to the application of a fast Fourier transform (FFT-) based Wiener deconvolution for various values of a regularization parameter $\lambda$.

Fig. 19 shows the reconstructed signal after using the FFT-based Wiener deconvolution for the various values of the regularization parameter $\lambda$.

Fig. 20 shows an example of a perfusion analysis result via perfusion kernels that is comparable to other patient angiograms and can therefore be leveraged for outcome prediction or diagnosis.

DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

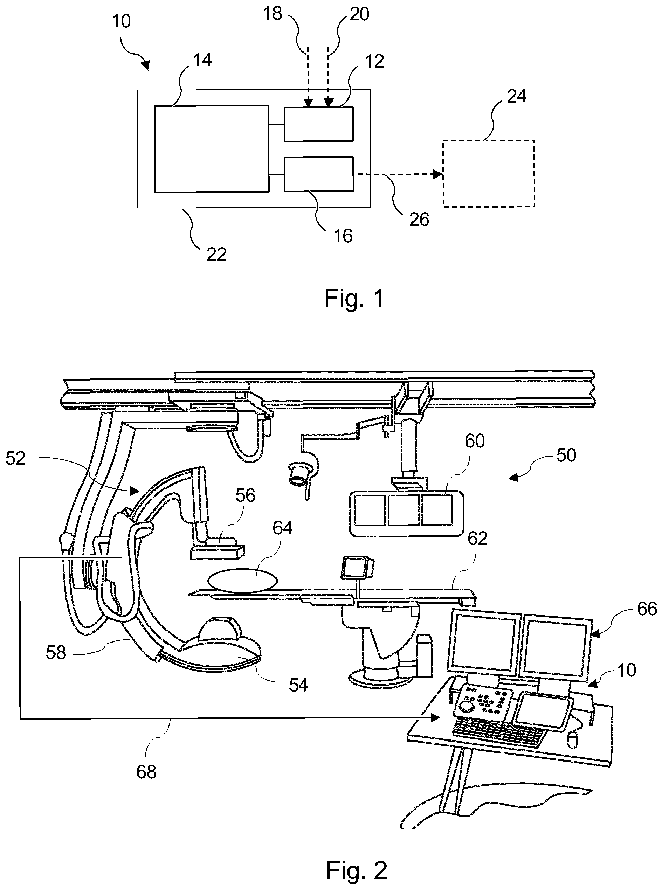

Fig. 1 schematically shows an example of a device 10 for peripheral perfusion analysis. The device 10 comprises a data input 12, a data processor 14 and an output interface 16. The data input 12 is configured to provide a sequence of 2D angiograms, indicated with a first input arrow 18. The data input 12 is also configured to provide a measured perfusion signal of perfused contrast agent in the region of interest, indicated with a second input arrow 20. The data processor 14 is configured to extract the region of interest. The data processor 14 is also configured to define a blood supply section comprising blood flow entry into and blood flow exit out of the region of interest. The data processor 14 is further configured to segment the blood supply section to extract regions occupied by detectable vessels. The data processor 14 is furthermore configured to identify vessels responsible for an inflow to extract the blood inlet into the region of interest. The data processor 14 is also configured to sum up all blood inlet of the section providing a perfusion sum. The data processor 14 is also configured to deconvolve the perfusion signal of the region of interest with the perfusion sum. The output interface 16 is configured to provide the deconvolution result.

Shown as an option in Fig. 1, a display 24 is provided. An output arrow 26 indicates the provision of display data from the output interface 16. The deconvolving comprises generating an outcome in form of at least one of the group of parameter and curves. The outcome is provided for clinical evaluation. In an option, the outcome is displayed on the display 24.

The data input 12, the data processor 14 and the output interface 16 can be provided within a common structure, like a housing, indicated with a frame 22, or as separately arranged components.

The data input 12 can also be referred to as data input module. The data processor 14 can also be referred to as data processing module. The output interface 16 can also be referred to as output interface module. The data input 12 can also be referred to as data supply, as image supply, as image data supply, as input unit or simply as input.

In an example, the data input 12 is data-connectable to an imaging source arrangement like an X-ray system, e.g. a C-arm, providing the 2D image data of the subject which is used for the information retrieval steps.

In an example, the image data input 12 is data-connectable to a data storage having stored the 2D image data.

The data processor 14 can also be referred to as data processing arrangement, as processor unit or as processor. In an example, the data processor 14 is data-connected to the image data input and the output interface.

The output interface 16 can also be referred to as output or output unit. In an example, the output interface 16 is data-connectable to a display arrangement or display device, e.g. the display 24. In another example, the output interface 16 is data-connected to the display 24.

In an example, not further shown in Fig. 1, by the deconvolving, the measured contrast density signals are standardized to perfusion kernels.

In an example, also not further shown in Fig. 1, for the identification, inflow and outflow of blood are determined for the detected vessels.

In an example, not further shown in Fig. 1, the region of interest of the subject comprises at least a subregion of at least one of the group of a foot, hand and head of a subject. The region of interest of the subject thus relates to, i.e. belongs to a foot, hand or head.

In an example, also not further shown in detail, the identification of the vessels with the inflow extracts an arterial input function for the respective angiogram. Further, the AIF extraction is provided covering regions of the image plane within a foot silhouette, a hand silhouette or a head silhouette.

In an example, not further shown in Fig. 1, the AIF covers all inlets to the perfusion ROI. As an option, in addition or alternatively, the AIF ignores all outlets.

As another, alternative option, the AIF also covers at least a part of the outlets. In an option, all of the outlets are considered.

In an example, also not further shown in detail, the blood supply section is provided as a boundary layer enclosing an area as the region of interest.

In an example, not further shown in Fig. 1, the blood supply section is provided as a convex form enclosing the region of interest.

In an example, also not further shown in detail, starting from the defined blood supply section, growing sub-portions are determined and an approximation index is provided to reflect possible ambiguity due to an increasing distance of the perfusion parts from the blood supply section.

In an example, not further shown in Fig. 1, the blood supply section is provided orthogonal to a longitudinal direction of the tibia bone at the lower end of the tibia bone.

Fig. 2 shows an example of a medical imaging system 50. The medical imaging system 50 comprises an X-ray imaging device 52 configured for generating X-ray images of a region of interest. The X-ray images comprise contrast-injected images and non-injected images.

Further, the medical imaging system 50 comprises an example of the device 10 for peripheral perfusion analysis according to one of the preceding example. The X-ray imaging device 52 generates the X-ray images for the sequence of 2D angiograms.

As an example, the medical imaging system 50 is shown in the context of an operation room in a hospital, e.g. a cath lab. The X-ray imaging device 52 is shown as a C-arm device with an X-ray source 54 and an X-ray detector 56 mounted to opposite ends of a movably supported C-arm 58. For example, a ceiling mounted rail system is provided for suspending the C-arm. Further, a monitor arrangement 60 is indicated plus lighting equipment. A subject support 62, e.g. a table, is provided for arranging a subject 64 for X-ray imaging. A console 66 is shown in the foreground providing control of the operation of the medical imaging system 50. In an example, the console 66 comprises the example of the device 10 for peripheral perfusion analysis (not further shown in Fig. 2). A data connection line 68 indicates the provision of 2D X-ray images from the X-ray imaging device 52 to the console 66, i.e. the device 10 for peripheral perfusion analysis.

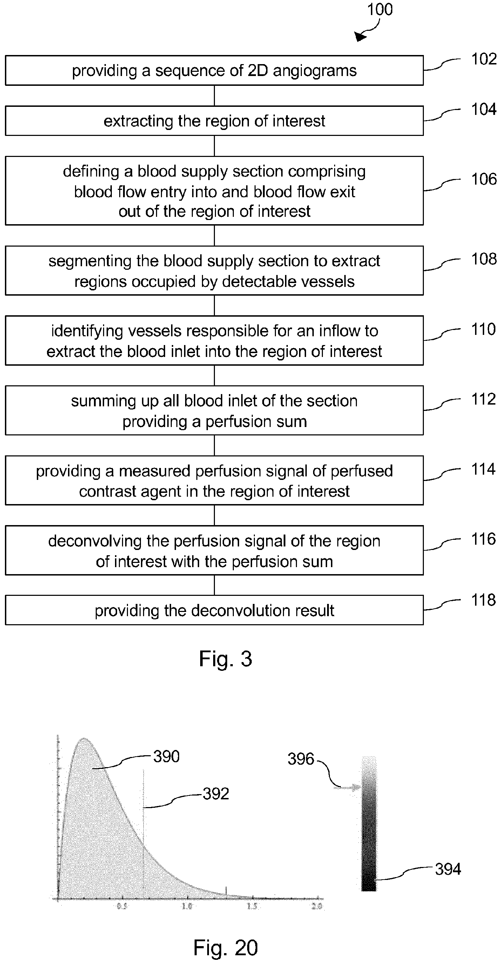

Fig. 3 shows steps of an example of a method 100 for peripheral perfusion analysis. The method 100 comprises the following steps: In a first step 102, a sequence of 2D angiograms is provided. In a second step 104, the region of interest is extracted. In a third step 106, a blood supply section comprising blood flow entry into, and blood flow exit out of the region of interest is defined. In a fourth step 108, the blood supply section is segmented to extract regions occupied by detectable vessels. In a fifth step 110, vessels responsible for an inflow are identified to extract the blood inlet into the region of interest. In a sixth step 112, all blood inlet of the section is summed up providing a perfusion sum. In a seventh step 114, a measured perfusion signal of perfused contrast agent in the region of interest is provided. In an eighth step 116, the perfusion signal of the region of interest is deconvolved with the perfusion sum. In a nineth step 118, the deconvolution result is provided.

The deconvolving can also be referred to as deconvolving.

In an example, the extracting the region of interest is also referred to as defining the region of interest.

In an example, the blood supply section is defined as a boundary region with a predetermined thickness.

In an option, the region of interest is defined/extracted first, and the blood supply section is defined as an adjacent boundary section.

In another option, the blood supply section is defined/extracted first, for example as a boundary section, or boundary layer, and the region of interest is defined as an adjacent region, i.e. a region contacting the blood supply section.

In an example of the method, by the deconvolving, the measured contrast density signals are standardized to perfusion kernels.

In another example of the method, not shown in detail, the deconvolving comprises generating an outcome in form of at least one of the group of parameter and curves. Further, the outcome is provided for clinical evaluation; wherein, preferably, the outcome is displayed.

In an example, for the 2D angiograms, a sequence of contrast-injected 2D X-ray images of the region of interest is provided and a non-contrast mask image of the region of interest is subtracted from each of the sequence of contrast-injected 2D X-ray images to generate the 2D angiograms.

The blood supply section can also be referred to as blood supply area or blood supply boundary or section of blood supply.

In a further example of the method, for the identifying, i.e. the identification, inflow and outflow of blood are detected, i.e. determined for the detected vessels.

The blood inlet can also be referred to as blood flow entries.

In an example of the method, not shown in detail, the identification of the vessels with the inflow extracts an arterial input function for the respective angiogram. Further, the AIF extraction is provided covering regions of the image plane within a foot silhouette, hand silhouette or head silhouette.

In an example of the method, the AIF covers all inlets to the perfusion ROI. As an option, the AIF ignores all outlets.

In an example, "ignored" means that they are not taken into account when computing the AIF, i.e. only the vessels are taken that feed the ROI. The outlets are not a subset of the blood supply section.

In another option, outputs, e.g. arterial, not venous, are not ignored., i.e. they are considered. As an example, the outlets are subtracted as they are bypassing the perfusion region of interest (not relevant for the ankle scenario though, because only veins are coming back).

In a further example, time delays between macrovascular inlets and outlets of a region of interest are taken into account by making use of the detected macrovascular connections within a region of interest, so that it is known for which inlet which (sum of) outlets passes through the region of interest without feeding it. As an option, (temporal) correlation between inlet and outlet curves is provided to identify the time shift and correcting for it before subtraction. This shift can be small with clinical frame rates in macro vessels and may only be measurably compromised in severely diseased patients.

In an example, the region for AIF extraction must only cover regions of the image plane within the foot silhouette.

In an example, the defined blood supply section is provided as an arterial input function for the perfused region of interest.

The defining can also be referred to as selecting the blood supply section. In an example of the method, the blood supply section is provided as a boundary layer enclosing an area as the region of interest.

In an example of the method, the blood supply section is provided as a convex form enclosing the region of interest.

As an example, the blood supply section is provided having a shape of one of a circle, oval or elliptic form, square, rectangular and polygonal form.

In an example of the method, starting from the defined blood supply section, growing sub-portions are determined and an approximation index is provided to reflect possible ambiguity due to an increasing distance of the perfusion parts from the blood supply section.

The sub-portions can also be referred to as sub-volumes or sub-quantities or subparts.

In an example of the method, the blood supply section is provided orthogonal to a longitudinal direction of the tibia bone at the lower end of the tibia bone.

In an example, the sub-portions are provided in a growing manner in the distal direction.

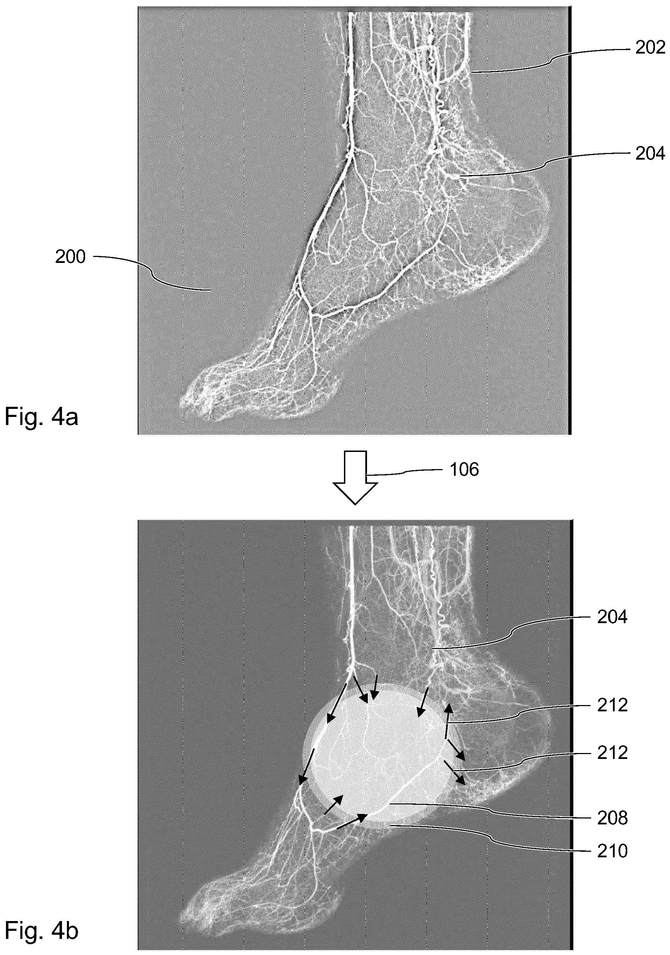

Fig. 4a and Fig. 4b show a first example of an extraction of an arterial input function.

Fig. 4a shows an option for an arterial input function region extraction. Fig. 4a shows a 2D X-ray perfusion image 200 of an anatomical structure 202, e.g. a foot. The 2D X-ray perfusion image 200 shows a vasculature structure 204 within a silhouette of the foot. An arrow 206 indicates to provide a mask for macrovascular candidates.

Fig. 4b shows as an example that an AIF region 208 for arbitrary user-defined perfusion regions of interest (ROIs) can be found by morphologically dilating the user-defined ROI yielding a thin marginal region 210 for the AIF extraction (patterned). Prior anatomical knowledge about the inlet flow direction is thus not exploited. Rather, inlet extraction needs to compare detected flow gradients of vessel fragments within the marginal region 210 with reference vectors 212 normal to the perfusion ROI 210 outer border (determined e.g. by fitting a cubic spline the edge pixels of the ROI).

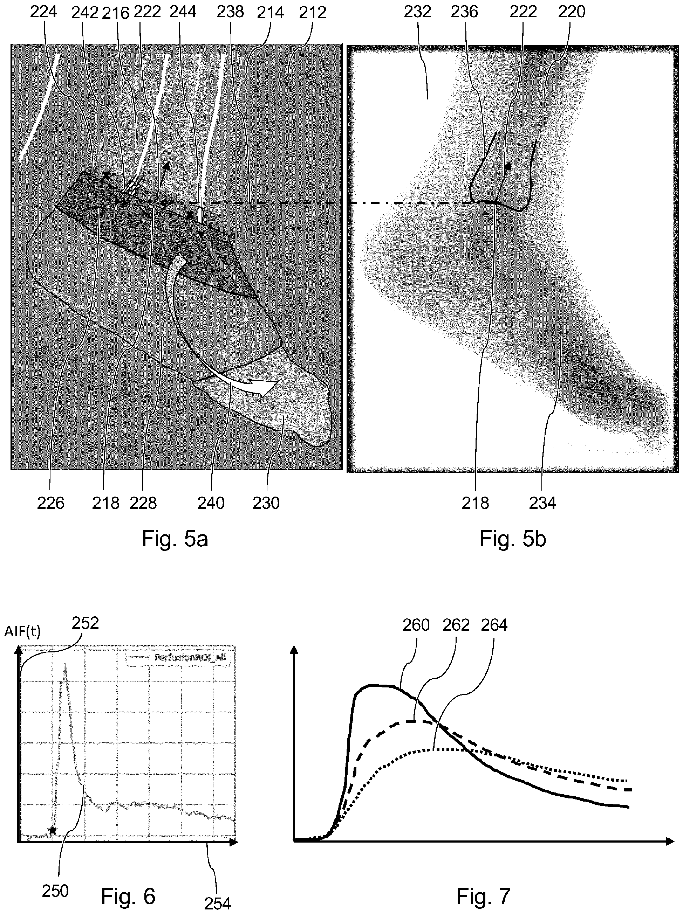

Fig. 5a and Fig. 5b show a second example of an extraction of an arterial input function.

Fig. 5a shows a 2D X-ray perfusion image 212 of an anatomical structure 214, e.g. a foot. The 2D X-ray perfusion image 212 shows a vasculature structure 216 within a silhouette of the foot. Fig. 5a shows an option for an arterial input function region extraction. Exploiting knowledge about the foot anatomy, the module detects a reference point 218 at the condyles of the lower end of the tibia bone and the pose of the tibia first (cross and arrow 222) and then constructs an AIF region (patterned region) as a small stripe 224 orthogonal to the tibia and masked by the foot silhouette. Appropriate perfusion regions 226, 228, 230 (further patterned, e.g. dotted or shadowed, regions), for which the AIF fulfils all necessary criteria, can be generated by morphologically growing regions within the foot silhouette, seeded at the AIF region. Resulting perfusion kernels after a deconvolution can be displayed for different growth states.

Fig. 5b shows an X-ray image 232 of a foot 234 for identifying the lower end of the tibia bone, indicated with a contour line 236. A dotted line 238 indicates the spatial transfer of the location from the X-ray image 232 to the perfusion image 212.

The stripe 224 is provided for identifying vessel segments 242, 244 in this region or layer that supplies the perfusion region(s), as indicated with small supply arrows.

A twisted arrow 240 indicates the morphologically growing of the regions within the foot silhouette.

Fig. 6 shows an example of a graph with a curve 250 of determined perfusion in the selected region of interest of Fig. 5a. A vertical axis 252 indicates the related arterial input function and a horizontal axis 254 indicates time.

Fig. 7 shows a graph with different curves for different kernels for the region of interest. A first curve 260 indicates the kernel of the first region of interest 226 of Fig. 5a; a second curve 262 indicates the kernel of the second region of interest 228 of Fig. 5a; and a third curve 264 indicates the kernel of the third region of interest 226 of Fig. 5a.

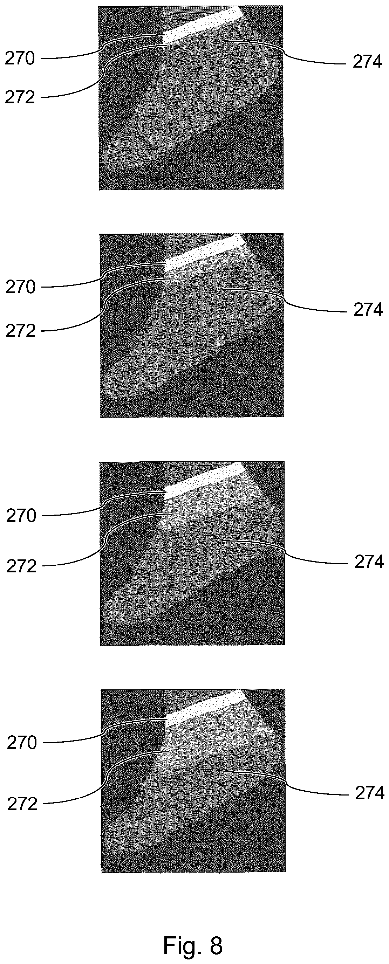

Fig. 8 shows an example of growing valid perfusion regions of interest for the example of Fig. 5a, i.e. for the second option of the AIF region extraction. A first region of interest 270 is indicated with a bright grey value; a second region of interest 272 is indicated with a middle grey value; and a third region of interest 274 is indicated with a darker grey value.

In the top part of Fig. 8, the first region of interest 270 is provided as a band. The second region of interest 272 is provided as a rather small band. The remaining region, i.e. the third region of interest 274 is rather large.

In the second part from the top of Fig. 8, the first region of interest 270 is again provided as a band. The second region of interest 272, however, is also provided as a small band. The remaining region, i.e. the third region of interest 274 is still quite large.

In the third part from the top of Fig. 8, the first region of interest 270 is again provided as a band. The second region of interest 272, however, is provided larger, extending from the band of the first region of interest 270. The third region of interest 274 is still large.

In the bottom part of Fig. 8, the first region of interest 270 is still provided as a band. The second region of interest 272, however, is provided as a large area, and the third region of interest 274 is a bit smaller.

Thus, growing regions are provided. As an example, they are treated differently, since the further away from the supply region, the more ambiguous the interpretation of perfusion signals relating to the supply region.

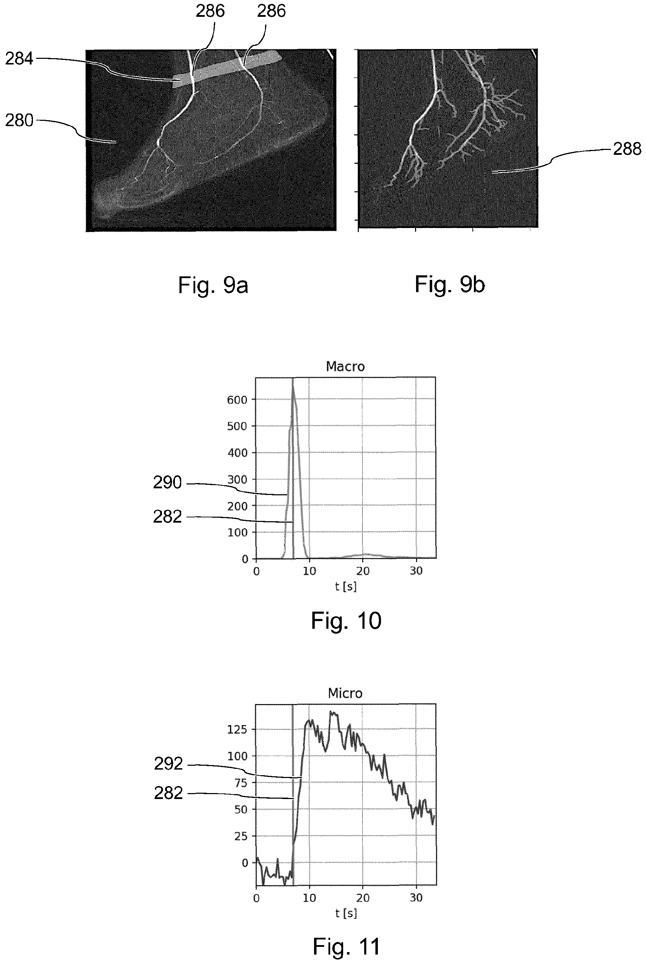

Fig. 9a shows a first example of a mask image for a perfusion image shown in Fig. 9b. Fig. 9a shows exemplary segmentations of the vasculature: As an example, a single DSA frame 280 at the time as marked with a vertical bar 282 in Fig. 10 and Fig. 11 is provided. An AIF region 284 is marked in a first pattern and contains the macrovascular parts indicated with reference numerals 286. A macrovascular segmentation 288 is provided on the full frame-time as marked by the vertical bar 282. Macrovascular response 290 within the AIF (see Fig. 10) and microvascular response 292 for the rest of the foot (see Fig. 11) is indicated.

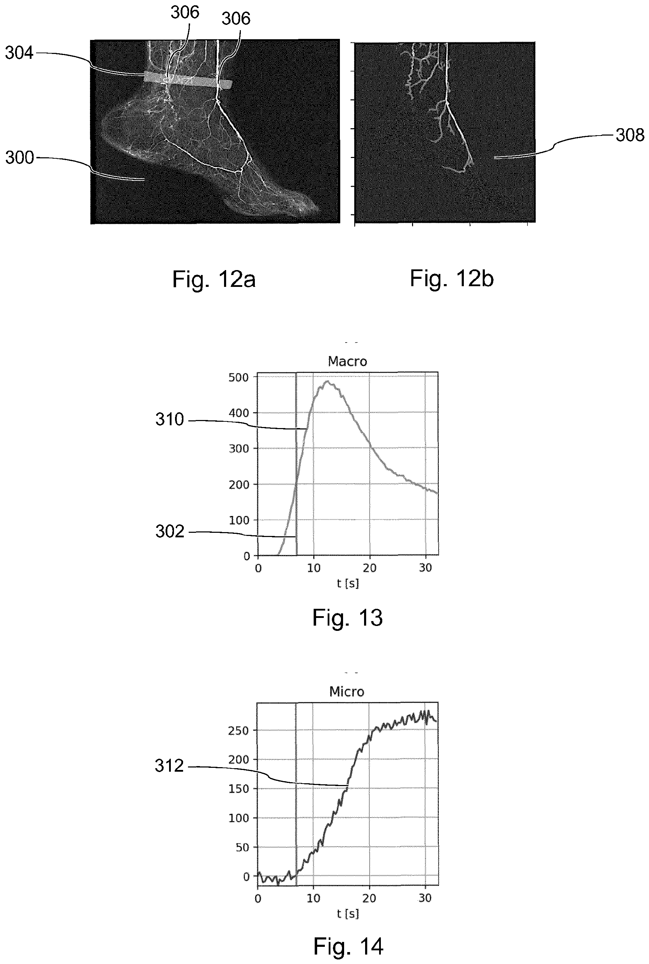

Fig. 12a shows exemplary segmentations of the vasculature: As an example, a single DSA frame 300 at the time as marked with a vertical bar 302 in Fig. 13 and Fig. 14 is provided. An AIF region 304 is marked in a first pattern and contains the macrovascular parts indicated with reference numerals 306. A macrovascular segmentation 308 is provided on the full frame-time as marked by the vertical bar 302. Macrovascular response 310 within the AIF (see Fig. 13) and microvascular response 312 for the rest of the foot (see Fig. 14) is indicated.

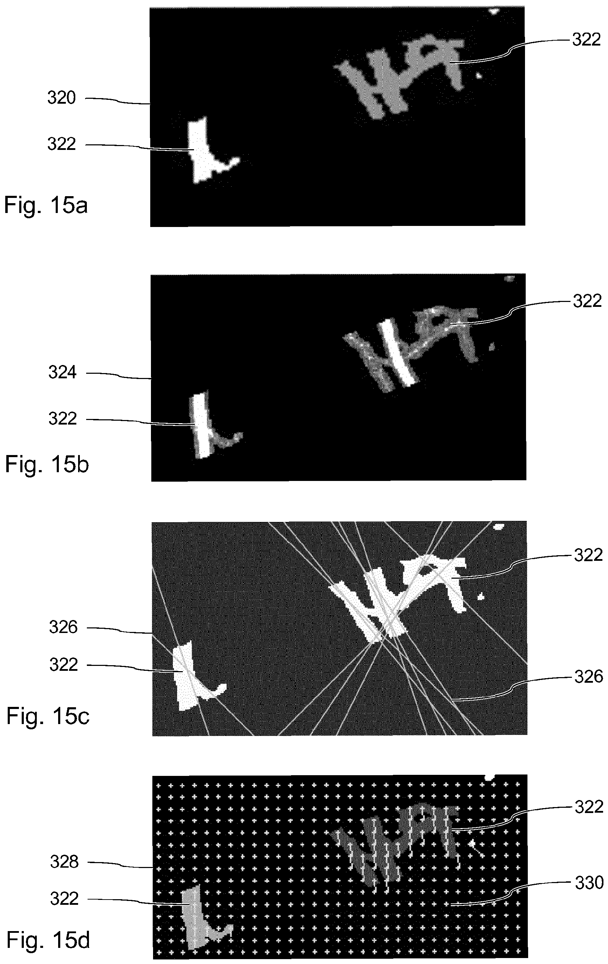

Fig. 15a shows an example of an inlet extraction module for the example of Fig. 9a as binary mask 320 with components.

Fig. 15b shows a digital subtraction angiography (DSA) maximum intensity projection (MIP) image relating to Fig. 15a.

Fig. 15c shows a Hough transform being used to detect lines, i.e. vessel components within the binary overall mask.

Fig. 15d shows an example for each of the final components being subject to an individual comparison with a reference flow vector to detect the set of inlet components.

Using the time as a third dimension, connected components 322 of the macrovasculature can be detected within the AIF region (example for Figs. 9a, 9, 12a, 12b as binary mask with four components, as shown in Fig. 15a.

Fig. 15b shows a DSA MIP image 324 masked by the binary mask of Fig. 15a. Due to the projective nature of 2D angiography, vessels of different flow components may still overlap due to the loss of depth information, i.e. form a single connected component.

Fig. 15c shows that a Hough transform can be used to detect lines 326, i.e. vessel components within the binary overall mask. Each line indicates one peak in the Hough space, filtering this set of lines to the most dissimilar ones, and assigning mask pixels to the closest line, can therefore separate even apparently connected flow components).

Fig. 15d shows that each of the final components will be subject to an individual comparison 328 with a reference flow vector 330 to detect the set of inlet components. The component-wise flow vector can be approximated by the average optical flow response in the corresponding region (right).

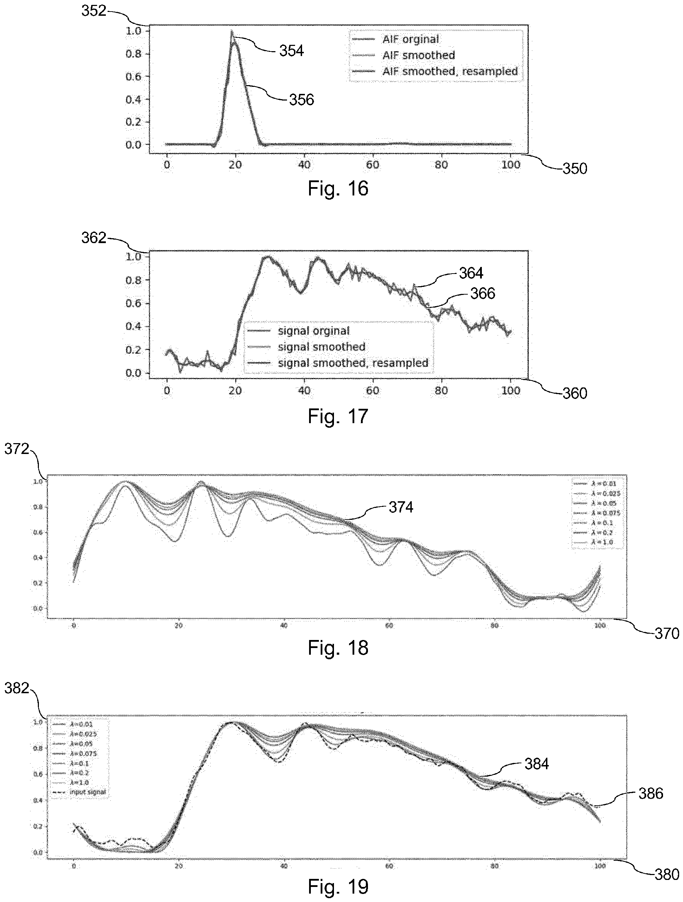

Fig. 16 shows an example of a graph indicating the AIF in a deconvolution process. A horizontal axis 350 indicates the time with the number of frames. A vertical axis 352 indicates the AIF. A first curve 354 relates to an original AIF signal during pre-processing. A second curve 356 relates to a smoothened and resampled AIF signal during pre-processing.

Fig. 17 shows a graph indicating the perfusion signal as well as the perfusion kernel. A horizontal axis 360 indicates the time with the number of frames. A vertical axis 352 indicates the AIF. A first curve 364 relates to an original AIF signal during pre-processing. A second curve 366 relates to a smoothened and resampled AIF signal during pre-processing.

Fig. 18 shows curves relating to the application of a fast Fourier transform (FFT-) based Wiener deconvolution for various values of a regularization parameter $\lambda$. A horizontal axis 370 indicates the time with the number of frames. A vertical axis 372 indicates the estimated kernel vs. a maximum estimated kernel. A plurality of curves 374 is shown with parameter $\lambda$ values of e.g. 0.01 or 0.025 or 0.05 or 0.075 or 0.1 or 0.2 or 1.0.

Fig. 19 shows the reconstructed signal after using the FFT-based Wiener deconvolution for the various values of the regularization parameter $\lambda$. A horizontal axis 380 indicates the time with the number of frames; a vertical axis 382 indicates the signal scaled-shifted to 0,1. Again, a plurality of curves 384 is shown with parameter $\lambda$ values of e.g. 0.01 or 0.025 or 0.05 or 0.075 or 0.1 or 0.2 or 1.0. Further, also an input signal curve 386 is shown.

Figs. 16 to 19 show an example of a deconvolution process. The plots show the AIF and the perfusion signal as well as the perfusion kernel and the reconstructed signal after using the FFT-based Wiener deconvolution (as an example) for various values of the regularization parameter $\lambda$ (lambda).

Fig. 20 shows that an illustration of the perfusion analysis result via perfusion kernels is comparable to other patient angiograms and can therefore be leveraged for outcome prediction or diagnosis. A curve 390 is provided with an indicator 392 for an individual measurement. A scale 394 (healthy vs. diseased) is provided with a mark 396.

In an example, deconvolution techniques assume a linear relationship between the AIF and the perfusion signal in a ROI. If this assumption is held for the whole vascular system, a perfusion kernel could be determined for a region even for an AIF with missing inlet components (at the price that the perfusion kernel is then defined with respect to this incomplete AIF). However, since non-linear effects are likely to occur in the overall vascular system, it is assumed that no relevant components are missing in the AIF and focus only on ROIs in the foot being not too far separated from the AIF region such that the linearity assumption can be made.

It is provided to analyze a region supplied with blood by several vessels across a certain section. It is thus possible to rely on 2D image data, instead of 3D projection data.

Macrovascular perfusion can be detected in angiography with eyesight, but there is also macrovascular perfusion related information beyond the resolution limit of digital subtraction angiography. For example, there is microvascular perfusion, e.g. capillaries, arterioles and venules.

In an example, for AIF extraction, an angiogram as a sequence of 2D X-ray images (frames) is provided. Further, a user defined region-of-interest (ROI) for perfusion analysis is provided. The proposed algorithm first determines a suitable AIF region (e.g. in an AIF region extraction module). Second, the segmentation module extracts regions in each frame which are occupied by the major vessels (as opposed to microvasculature the vessel net of which cannot be resolved by the angiogram resolution). Third, the inlet extraction module distinguishes vessels responsible for the inflow from those which are responsible for the outflow. Finally, the deconvolution-and-analysis module computes the standardized perfusion kernel according to one of a multitude of established methods and possibly extracts parameters from this curve. The curve and parameters can then be presented to the user for clinical evaluation. Since parameters are comparable (per region) across patients, they can be illustrated within the population context, e.g. by using an individual marker within a population distribution or a color bar that maps the closeness of the parameter to that of a diseased sub-population to a color.

In an example, during a perfusion related intervention, blockages or narrowing's in upstream vessels are identified and treated such that the blood supply to the foot improves, where symptoms to dysfunction perfusion, and subsequently ischemia and hypoxia show first. Although the vascular system of the foot does typically not change in morphology due to treatment, the system's behavior however does. After the intervention, different input pathways for perfusing the foot are balanced and used in a way that differs from the pre-interventional setting. This in turn changes the proportion and timing of blood reaching different sub-regions of the foot and thus the measured dynamics. In addition, these dynamics will also depend on the contrast injection protocol. The measured contrast density signals are therefore normalized with respect to the arterial input signal to the foot which is typically formed by a superposition of the supply from several arteries, e.g. ATA (anterior tibial artery), PTA (posterior tibial artery) or PEA (peroneal artery). In particular for severely diseased critical limb ischemia (CLI) patients, even these inlet characteristics can be quite complex due to a morphologically changed vascular tree, total occlusions, neovascularization and collaterals, the proposed solution addresses this.

In an example, an automated extraction of AIF regions dedicated to each selected perfusion ROI can be easily tested by deconvolving the raw perfusion ROI signal as it is readily available with the perfusion kernel provided by the algorithm. When using a global AIF, the signal would not vary over different ROI selections.

The proposed solution can be applied for any type of perfusion analysis from MRA, CTA or X-ray angiography as at least parts of it can be generalized to 3D perfusion analysis. In particular, the solution is dedicated to 2D peripheral angiograms and further particular for perfusion analysis based on interventional data.

The proposed solution is an enabler for comparing extracted perfusion characteristics within one patient or between patients and is therefore imperative when aiming for the prediction of clinical endpoints such as long-term treatment outcome. The standardization of perfusion measurements achieved by this solution is also a key enabler for correlating angiography based perfusion measurements with other measurement approaches of hemodynamic properties that do not rely on contrast media, e.g. iPPG (imaging photoplethysmography).

By considering the feeding, i.e. input region, for the region of interest, also confounding factors can be considered for TDC shape. An example for an impact that is taken into account by considering the feeding region is the contrast injection protocol, i.e. bolus volume and injection rate, or the changes to the injected bolus wave while it passes through the leg - including the ATK/BTK (above-the-knee/below-the-knee) lesion (bolus splitting up at bifurcations into several components, as a function of the lesion/intervention, which may be differently delayed before reaching the foot, resulting in so-called contrast races. Even though these confounding factors hinder valid comparisons between cases of pre- and post-interventional recordings and also comparisons to other patients when setting a current measurement into the context of a patient population, the proposed solution overcomes this at least partly.

By known deconvolution techniques, it is possible to "normalize" the measured signal with respect to the input signal. More formally, a linear relationship between the measured input signal, the AIF associated with a certain region, and the measured signal $S_\Omega$ in a ROI Q is assumed in form of a convolution:

$$ S_\Omega(t) = \int_{t_0}^{t} d\tau \; k_\Omega(t - \tau) \, AIF(\tau) $$

with an unknown kernel $k_\Omega(t)$ encoding the impulse response of the perfused system and $t_0$ referring to the start time of the measurement. This kernel is called perfusion kernel in the present context. Deconvolution techniques allow for solving this equation for the unknown perfusion kernel. The deconvolved signal, i.e. the perfusion kernel, is independent of factors that had shaped the input signal before it actually entered the perfusion region of interest - the foot in this case - and encodes the perfusion characteristics of the ROI $\Omega$.

In an example, as a start it is provided that the user ROI boundary does not intersect with any inlets/outlets. The user is either notified or for finding a suitable supply region, the user ROI is provided for region-grow the ROI until inlets are included. As an option, the TDC can be computed from the smaller ROI, but the supply region would be extracted as the boundary of the enlarged ROI.

In an example, not shown in detail, the steps of region extracting, segmenting, extracting inlet are provided by modules. As an example, an inlet extraction module is provided. Independent from the approach, which is used to extract the AIF region, the macrovascular components lying within this region may contain arteries and veins conducting blood into different directions. The inlet extraction module separates inflow from outflow to the desired perfusion region and thus ensures a valid AIF.

In an example, it is distinguished between venous and arterial (i.e. bypassing) outlets via the macrovascular connectivity in the region of interest, which is also detected. This split as mentioned above is suitable in cases where only the microvascular parts of the ROI curve are deconvolved.

As a further example, an AIF region extraction module is provided. Given an angiogram of the foot, this module identifies suitable AIF regions for a given perfusion ROI that fulfils above criteria. The invention proposes two different options as explained further above; see also Figs. 4a and 4b and Figs. 5a and 5b.

As another example, a segmentation module is required to segment the macrovasculature for each frame in the DSA sequence. Using this micro/macro split, the microvascular and macrovascular TDC can be computed as input for the AIF (partial macrovascular response in the constructed AIF region). Depending on the clinical application, one may either take the total TDC in the selected perfusion ROI as the signal to be de-convolved or its micro-/macrovascular component. In the latter case, this segmentation module is used to separate the two components also for the perfusion ROI. Figs. 10 and 11 as well as Figs. 13 and 14 show examples for the macrovascular response in the AIF region (Fig. 11 and Fig. 13) as well as the microvascular response in the rest of the foot (Fig. 12 and Fig. 14). The vertical bar marks the time for which the frames on the left are plotted. Macrovascular segmentation is shown in straight lines. The segmentation module can be realized by data driven segmentation algorithms, e.g. that are trained on DSA MIPs and vessel segmentation ground truth thereof. The model is transferred to single frames during deployment, since getting manual ground truth on a per-frame basis is not feasible.

In a first option, for arbitrary, user-defined perfusion ROIs as illustrated in Fig. 4a, a valid AIF region is constructed by morphologically dilating the perfusion ROI to obtain a small marginal ROI for extracting the AIF. Since there is no single reference vector for inflow vector comparisons, reference vectors must be locally computed taking the outer contour of the perfusion ROI into account. In an example, the outer edge of the ROI is detected, e.g. using Canny edge filters, and a cubic spline contour is fitted through the edge points. For each point on this spline, a vector orthogonal to the contour can be computed and used as a local reference vector for determining the inlet components.

In a second option, another approach is provided: A possible choice for an AIF region in peripheral angiograms is the ankle region, where all major vessels enter the foot. As illustrated in Fig. 5a, such a ROI can be constructed by first detecting the condyles at the lower end of the tibia as well as a directional vector resembling the longitudinal extent of the tibia bone. This can be achieved by standard template matching algorithms or using data-driven learning algorithms that are trained for landmark or contour detection. Given this reference point and the directional vector, a ROI can be constructed by generating a small stripe of pixels as a thickened line orthogonal to the directional vector. The stripe-like region is limited by a binary mask of the foot silhouette which can also be extracted by one of a multitude of established 2D segmentation algorithms.

A specialty of this option is that it exploits anatomical prior knowledge as it can use the directional vector directly as the single reference vector for the arterial inflow at the ankle with which detected candidate flow gradients can be compared.

Valid perfusion ROIs can be constructed by growing regions using morphological dilation (cf. Fig. 8) within the foot silhouette, seeded at the AIF stripe. This limits the scope of perfusion regions for which this approach can be used when comparability to other patients should be maintained. It also assumes that there is no retrograde backflow from the forefoot and may therefore be only an approximation for some heavily diseased patients.

It is noted that the technique can also be used with arbitrary perfusion ROIs, in case the analysis is limited to a single patient (e.g. pre- versus post-interventional comparison). In this case a real impulse response of the system is not computed, but that of a virtually not existing system (as components of the AIF inflow may never reach the selected perfusion ROI). Comparisons within a single patient are however still valid if the interventional therapy does not directly impact the foot region and a linearity assumption holds for the inflow, i.e. the outflow magnitude of sub-components of the AIF (e.g. ATA, PTA, peroneal) change linearly with the overall input (e.g. if the overall input is doubled, all sub-vessel components also double).

A first step uses the binary macro-vasculature map over time and extracts connected components that fall within the AIF region. Fig. 15a illustrates the components plotted as an MIP over time. This yields a set of binary sub-masks (Fig. 15b), one per component to be investigated. Due to the projective nature of 2D angiography, vessels of different flow direction may still overlap in the 2D imaging plane due to the loss of depth information. They form a single connected component although they would be separate in 3D world coordinate space and/or even show flow in different directions.

Hough transform can be used to detect lines (cf. Fig. 15c), i.e. vessel sub-components within the binary overall mask: Each line indicates one peak in the Hough space. Filtering the set of lines in the Hough space to the most dissimilar candidates yields a set of abstract vessel prototypes. Each mask pixel can be assigned to the closest line prototype to form as many sub-mask components as there are lines in the set. Therefore, even apparently connected flow components can be separated.

Each of the final components will then be subject to an individual comparison with a reference flow vector to detect the set of inlet components. The component-wise flow vector can be approximated by the average optical flow response in the corresponding region (cf. Fig. 15d) after spatially smoothing the DSA sequence. If the fluoroscopy frame rate is too low compared to the contrast medium flow velocity for accurately determining the local flow direction, video frame interpolation techniques may be utilized as a pre-processing step.

The component-wise flow vectors are compared to the local reference flow vector by using the inner product and testing for a certain angle range that excludes outward or tangential flow.

After constructing the AIF signal by superposition of the relevant single vessel components, the measured perfusion signal can be deconvolved based on any of the established regularized deconvolution methods (SVD-based, FFT-based, iterative etc.). The resulting perfusion kernel can then be used to qualitatively judge perfusion or to extract parameters after fitting a perfusion model such as a gamma variate curve.

The latter fit will be more appropriate after deconvolution, since the signal to be fitted does not consist of a set of delayed components anymore, the superposition of which could result in a curve shape far away from the model and where the model fit error may strongly depend on the disease status. Fig. 16 shows an example AIF, the perfusion signal (Fig. 17) as well as the perfusion kernel (Fig. 18) and the reconstructed signal after using an FFT-based deconvolution (Fig. 19).

From this example, one can infer that several parameters being characteristic for the perfusion of the selected ROI can be derived from the estimated perfusion kernel. In particular, the peak time can be used as a standardized measure for the time delay in the perfusion of the AIF region and the perfusion ROI. Similarly, the time at which the perfusion kernel has decayed to half of its maximum could serve as a measure for the wash-out time scale. The mean of the (absolute value of the) perfusion kernel over time could serve as a standardized measure of the perfusion intensity of the given ROI. Using the AIF region extraction first option, such parameters could be evaluated for the whole foot by a sliding window approach with respect to the perfusion ROI. The result may then be visualized as colored overlays on the angiography.

Since parameters are comparable (per perfusion region) across patients, they can be illustrated within the population context, e.g. by using an individual marker within a population distribution or a color bar that maps the closeness of the parameter to that of a diseased sub-population to a color (see Fig. 20).

The first option may be referred to as a general approach. The first option may be referred to primary option; the second option as secondary option.

The term "subject" may also be referred to as individual. The "subject" may further also be referred to as patient, although it is noted that this term does not indicate whether any illness or disease is present with the subject.

In an example, a computer program or program element for controlling an apparatus according to one of the examples above is provided, which program or program element, when being executed by a processing unit, is adapted to perform the method steps of one of the method examples above.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it, which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device (10) for peripheral perfusion analysis, comprising:

   - a data input (12);
   - a data processor (14); and
   - an output interface (16);
   wherein the data input is configured to receive a sequence of 2D angiograms (18); and to receive a measured perfusion signal (20) of perfused contrast agent in the region of interest;
   wherein the data processor is configured to extract the region of interest; to define a blood supply section comprising blood flow entry into and blood flow exit out of the region of interest; to segment the blood supply section to extract regions occupied by detectable vessels; to identify vessels responsible for an inflow to extract the blood inlet into the region of interest; and to sum up all blood inlet of the section providing a perfusion sum; to deconvolve the perfusion signal of the region of interest with the perfusion sum; and
   wherein the output interface is configured to provide the deconvolution result.

2. Device according to claim 1, wherein by the deconvolving, the measured contrast density signals are standardized to perfusion kernels.

3. Device according to claim 1 or 2, wherein for the identification, inflow and outflow of blood are determined for the detected vessels.

4. Device according to claim 1, 2 or 3, wherein the region of interest of the subject comprises at least a subregion of at least one of the group of a foot, hand and head of a subject.

5. Device according to one of the preceding claims, wherein the identification of the vessels with the inflow extracts an arterial input function (AIF) for the respective angiogram; and
   wherein the AIF extraction is provided covering regions of the image plane within a foot silhouette, hand silhouette or head silhouette.

6. Device according to one of the preceding claims, wherein the AIF covers all inlets to the perfusion ROI.

7. Device according to one of the preceding claims, wherein the blood supply section is provided as a boundary layer enclosing an area as the region of interest.

8. Device according to claim 7, wherein the blood supply section is provided as a convex form enclosing the region of interest.

9. Device according to one of the preceding claims, wherein starting from the defined blood supply section, growing sub-portions are determined and an approximation index is provided to reflect possible ambiguity due to an increasing distance of the perfusion parts from the blood supply section.

10. Device according to one of the preceding claims, wherein the blood supply section is provided orthogonal to a longitudinal direction of the tibia bone at the lower end of the tibia bone.

11. Device according to one of the preceding claims, wherein a display (24) is provided;

    wherein the deconvolving comprises generating an outcome in form of at least one of the group of parameter and curves; and
    wherein the outcome is provided for clinical evaluation and displayed on the display.

12. A medical imaging system (50) comprising:

    - an X-ray imaging device (52) configured for generating X-ray images of a region of interest; wherein the X-ray images comprise contrast-injected images and non-injected images; and
    - a device (10) for peripheral perfusion analysis according to one of the preceding claims;

    wherein the X-ray imaging device generates the X-ray images for the sequence of 2D angiograms.

13. A method (100) for peripheral perfusion analysis, comprising the following steps:

    - receiving (102) a sequence of 2D angiograms;
    - extracting (104) the region of interest;
    - defining (106) a blood supply section comprising blood flow entry into and blood flow exit out of the region of interest;
    - segmenting (108) the blood supply section to extract regions occupied by detectable vessels;

- identifying (110) vessels responsible for an inflow to extract the blood inlet into the region of interest;
- summing up (112) all blood inlet of the section providing a perfusion sum;
- providing (114) a measured perfusion signal of perfused contrast agent in the region of interest;
- deconvolving (116) the perfusion signal of the region of interest with the perfusion sum; and
- providing (118) the deconvolution result.

14. A computer program enabling a processor to carry out the method of claim 13.

15. A computer readable medium having stored the program element of claim 14.

Fig. 1

Fig. 2

100

| providing a sequence of 2D angiograms | 102 |

| extracting the region of interest | 104 |

| defining a blood supply section comprising blood flow entry into and blood flow exit out of the region of interest | 106 |

| segmenting the blood supply section to extract regions occupied by detectable vessels | 108 |

| identifying vessels responsible for an inflow to extract the blood inlet into the region of interest | 110 |

| summing up all blood inlet of the section providing a perfusion sum | 112 |

| providing a measured perfusion signal of perfused contrast agent in the region of interest | 114 |

| deconvolving the perfusion signal of the region of interest with the perfusion sum | 116 |

| providing the deconvolution result | 118 |

Fig. 3

390
392
396
394

Fig. 20

Fig. 4a

Fig. 4b

Fig. 5a

Fig. 5b

Fig. 6

Fig. 7

270

272

274

270

272

274

270

272

274

270

272

274

Fig. 8

Fig. 9a                    Fig. 9b

Fig. 10

Fig. 11

Fig. 12a　　　　Fig. 12b

Fig. 13

Fig. 14

EP 4 197 442 A1

Fig. 15a

Fig. 15b

Fig. 15c

Fig. 15d

21

352

1.0
0.8        354
0.6            356
0.4
0.2
0.0
     0    20    40    60    80    100    350

AIF orginal
AIF smoothed
AIF smoothed, resampled

Fig. 16

362

1.0
0.8            364
0.6              366
0.4
0.2
0.0
     0    20    40    60    80    100    360

signal orginal
signal smoothed
signal smoothed, resampled

Fig. 17

372

1.0
0.8            374
0.6
0.4
0.2
0.0
     0      20      40      60      80      100    370

λ = 0.01
λ = 0.025
λ = 0.05
λ = 0.075
λ = 0.1
λ = 0.2
λ = 1.0

Fig. 18

382

1.0
0.8
0.6            384
0.4              386
0.2
0.0
     0      20      40      60      80      100    380

λ = 0.01
λ = 0.025
λ = 0.05
λ = 0.075
λ = 0.1
λ = 0.2
λ = 1.0
input signal

Fig. 19

Europäisches
Patentamt

European
Patent Office

Office européen
des brevets

# EUROPEAN SEARCH REPORT

Application Number

EP 21 21 4583

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LIEBESKIND ALEXANDER ET AL: "Automatic Estimation of Arterial Input Function in Digital Subtraction Angiography", 21 October 2019 (2019-10-21), COMPUTER VISION - ECCV 2020 : 16TH EUROPEAN CONFERENCE, GLASGOW, UK, AUGUST 23-28, 2020 : PROCEEDINGS; [LECTURE NOTES IN COMPUTER SCIENCE ; ISSN 0302-9743], SPRINGER INTERNATIONAL PUBLISHING, CHAM, PAGE(S) 393 - 402, XP047545404, ISBN: 978-3-030-58594-5 [retrieved on 2019-10-21] | 1,3-15 | INV. A61B6/00 |
| Y | * abstract * <br> * figures 1a, 5 * <br> * Section 2.1; page 395 * <br> * page 394 * <br> * page 397 - page 398 * <br> ----- | 2 | |
| X | KAO YI-HSUAN ET AL: "Automatic measurements of arterial input and venous output functions on cerebral computed tomography perfusion images: A preliminary study", COMPUTERS IN BIOLOGY AND MEDICINE, NEW YORK, NY, US, vol. 51, 9 May 2014 (2014-05-09), pages 51-60, XP028876168, ISSN: 0010-4825, DOI: 10.1016/J.COMPBIOMED.2014.04.015 * abstract * <br> * page 51 - page 55 * <br> * figures 2, 3 * <br> ----- <br> -/-- | 1,3-6, 12-15 | TECHNICAL FIELDS SEARCHED (IPC) <br><br> A61B |

The present search report has been drawn up for all claims

| Place of search | Date of completion of the search | Examiner |
|---|---|---|
| Munich | 25 May 2022 | Ordavo, Ivan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

**EUROPEAN SEARCH REPORT**

Application Number

EP 21 21 4583

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | A.A. KONSTAS ET AL: "Theoretic Basis and Technical Implementations of CT Perfusion in Acute Ischemic Stroke, Part 1: Theoretic Basis", AMERICAN JOURNAL OF NEURORADIOLOGY, vol. 30, no. 4, 6 March 2009 (2009-03-06), pages 662-668, XP055604459, US ISSN: 0195-6108, DOI: 10.3174/ajnr.A1487 * page 666 - page 667 * | 2 | |

TECHNICAL FIELDS
SEARCHED     (IPC)

The present search report has been drawn up for all claims

| Place of search | Date of completion of the search | Examiner |
|---|---|---|
| Munich | 25 May 2022 | Ordavo, Ivan |

EPO FORM 1503 03.82 (P04C01)

**REFERENCES CITED IN THE DESCRIPTION**

**Patent documents cited in the description**

- WO 2005104936 A1